# EUROPEAN PATENT APPLICATION

(11) **EP 4 173 626 A1**
(43) Date of publication of application: **03.05.2023**
(21) Application number: 21205486.0
(22) Date of filing: 29.10.2021
(51) Int. Cl.: A61K 31/7024, A61P 9/10, A61P 11/00

(54) **PENTAGALLOYL GLUCOSE (PGG) FOR USE IN TREATMENT OF PULMONARY HYPERTENSION**

(71) Applicant: Charité - Universitätsmedizin Berlin, 10117 Berlin (DE)
(72) Inventor: Kübler, Wolfgang, 10965 Berlin (DE); Kucherenko, Mariya M., 10629 Berlin (DE); Knosalla, Christoph, 12203 Berlin (DE)
(74) Representative: Gulde & Partner

(57) **Abstract**

The present invention is directed to pentagalloyl glucose (PGG), a pharmaceutically acceptable salt thereof or a pharmaceutical composition comprising the same for use in treatment or prevention of pulmonary hypertension, preferably of pulmonary hypertension secondary to left heart disease.

## Description

### BACKGROUND OF THE INVENTION:

About 1% of the adult population suffers from pulmonary hypertension (PH). PH due to left heart disease (PH-LHD) (WHO group 2) is the most common form of PH, accounting for 65-80% of cases.

The characteristic feature of pulmonary hypertension (PH) is increased blood pressure in the pulmonary arteries. This is basically an increase in the pulmonary arterial mean pressure (mPAP) to greater than 25mmHg at rest. Morbidity and mortality of affected patients increases with the degree of PH. In the long term, PH can lead to strain on the heart muscle, causing the right ventricle in particular to dilate and lose strength, so that it can ultimately no longer transport the necessary amount of blood. Typical signs of pulmonary hypertension are narrowed pulmonary arteries with a thickened vascular wall. As a direct consequence, the oxygen supply to the body is reduced and the performance of those affected is drastically limited. In advanced stages, pulmonary hypertension can develop into a life-threatening condition.

The diagnosis of idiopathic pulmonary hypertension is very rare. Much more often, PH is the result of a pre-existing condition, such as left heart disease or lung disease and/or lack of oxygen (hypoxia). Symptoms of PH include shortness of breath, weakness, severe circulatory problems and eventually right heart failure. Patients who develop PH usually die of right cardiac decompensation.

Only a few drugs are currently approved for the treatment of pulmonary hypertension. Agents include prostanoids, sGC stimulators (riociguat), endothelin receptor antagonists and phosphodiesterase-5 inhibitors. However, the effectiveness of the drugs is low and the medications usually do not lead to a cure, but only delay the progression of the disease process. In addition, considerable side effects can occur, so that a lung transplantation is sometimes necessary instead of drug therapy. Importantly, these drugs are at present only approved for the relatively rare case of pulmonary arterial hypertension (including idiopathic pulmonary hypertension), whereas no pharmacological therapies are presently approved for treatment of the most frequent forms of PH, namely PH associated with left heart disease or lung disease and/or lack of oxygen.

Accordingly, the objective of the present invention is to provide an effective medical agent for treatment and prevention of pulmonary hypertension, in particular of pulmonary hypertension secondary to left heart disease.

### DESCRIPTION OF THE INVENTION:

The present invention is directed to subject matter as defined in the claims and as set out in detail below.

According to the present invention, pentagalloyl glucose (PGG) or a pharmaceutically acceptable salt thereof is used for treatment or prevention of pulmonary hypertension; preferably the pulmonary hypertension is secondary pulmonary hypertension; more preferably the pulmonary hypertension is pulmonary hypertension secondary to left heart disease (LHD; WHO group II).

It has surprisingly been found that administration of PGG - either as a free substance or coupled to a delivery vehicle like e.g. nanoparticles - is particularly suitable for the treatment of pulmonary artery stiffness in PH. The following findings demonstrate the efficacy and relevance of this invention. Firstly, the application of PGG in ex *vivo* cultured pulmonary arteries (PA) increased the elastin content in the pulmonary artery wall of PAs after treatment with elastase and increased the elasticity of pulmonary arteries after treatment with elastase. Secondly, *in vivo* application of PGG in the rat model of PH due to left heart disease leads to reduced right ventricular pressure load and right ventricular hypertrophy, to largely reversed pre-existing PH and right ventricular (RV) hypertrophy, to improved organisation of elastic fibres in the PA wall as well as to counteracted PA stiffening, and normalised PA biomechanics in vivo and ex vivo.

Thus, the inventors have shown that application of the elastin-stabilising compound PGG, either as a free substance or coupled to a delivery vehicle, improved significantly the biomechanical properties of the pulmonary artery, and is, therefore, able to normalise pre-existing haemodynamic changes and right heart strain in PH.

Pentagalloyl glucose (1,2,3,4,6-Penta-*O*-galloyl-β-D-glucose, PGG) is the pentagallic acid ester of glucose and, as such, is characterized by a D-glucose molecule esterified at all five hydroxyl moieties by gallic acid (3, 4, 5- trihydroxybenzoic acid). Thus, PGG includes the hydrophobic core of tannic acid as well as multiple phenolic hydroxyl groups, but does not possess the outer gallic acid residues and the hydrolyzable ester bonds associated with tannic acid. PGG is known to have stabilizing effects on elastin.

PGG for use of the present invention may be present in the form of a pharmaceutically acceptable salt. As used herein, the term "pharmaceutically acceptable salt" includes acid addition salts and base addition salts. Such salts may be formed by conventional means, for example by reaction of a free acid or a free base form of a compound of the invention with one or more equivalents of an appropriate acid or base, optionally in a solvent, or in a medium in which the salt is insoluble, followed by removal of said solvent, or said medium, using standard techniques (e.g. in vacuo or by freeze-drying). When the compounds of the present invention possess a free base form, the compounds can be prepared as a pharmaceutically acceptable acid addition salt by reacting the free base form of the compound with a pharmaceutically acceptable inorganic or organic acid, e.g. hydrohalides such as hydrochloride, hydrobromide, hydroiodide; other mineral acids and their corresponding salts such as sulfate, nitrate, phosphate, etc.; and alkyl- and monoarylsulfonates such as ethanesulfonate, toluenesulfonate and benzenesulfonate; and other organic acids and their corresponding salts such as acetate, tartrate, maleate, succinate, citrate, benzoate, salicylate and ascorbate. Further acid addition salts of the present invention include adipate, alginate, arginate, aspartate, benxenesulfonate (hesylate), bisulfate, bisulfite, bromide, butyrate, camphorate, camphorsulfonate, caprylate, chloride, chlorobenzoate, cyclopentanepropionate, digluconate, dihydrogenphosphate, dini- trobenzoate, dodecylsulfate, ethanesulfonate, fumarate, galacterate (from mucic acid), galacturonate, glucoheptaoate, glucorrate, glutamate, glycerophosplrate, hemisueci- nate, hemisulfate, heptanoate, hexanoate, hippurate, hydrochloride, hydrobromide, hydroiodide, 2-hydroxyethanesulfonate, iodide, isethionate, iso-butyrate, lactate, lactobionate, malate, malonate, mandelate, metaphosphate, methanesulfonate, methylbenzoate, monohydro- genphosphate, 2-naphthalenesulfonate, nicotinate, nitrate, oxalate, oleate, pamoate, pectinate, persulfate, phenylac- etate, 3-phenylpropionate, phosphate, phosphonate and phthalate. When the compounds of the present invention possess a free acid form, a pharmaceutically acceptable base addition salt can be prepared by reacting the free acid form of the compound with a pharmaceutically acceptable inorganic or organic base. Examples of such bases are alkali metal hydroxides including potassium, sodium and lithium hydroxides; alkaline earth metal hydroxides such as barium and calcium hydroxides; alkali metal alkoxides, e.g. potassium ethanolate and sodium propanolate; and various organic bases such as ammonium hydroxide, piperidine, diethanolamine and N-methylglutamine. Also included are the aluminum salts of the compounds of the present invention. Further base salts of the present invention include copper, ferric, ferrous, lithium, magnesium, manganic, manganous, potassium, sodium and zinc salts. Organic base salts include salts of primary, secondary and tertiary amines, substituted amines including naturally occurring substituted amines, cyclic amines and basic ion exchange resins, e.g., arginine, betaine, caffeine, chloroprocaine, choline, N,N'-dibenzylethylenediamine (benzathine), dicyclohexylamine, diethanolamine, diethylamine, 2-diethylaminoethanol, Z-dimethylami- noethanol, ethanolamine, ethylenediamine, N-ethylmorpholine, N-ethylpiperidine, glucamine, glucosamine, histidine, hydrabamine, iso-propylamine, lidocaine, lysine, meglumine, N -methyl-D-glucamine, morpholine, piperazine, piperidine, polyamine resins, procaine, purines, theobromine, triethanolamine, triethylamirre, trimethylamine, tripropylamine and tris-(hydroxymethyl)-methylamine (tromethamine).

Salts may also be prepared by exchanging a counter-ion of a compound of the invention in the form of a salt with another counter-ion, for example using a suitable ion exchange resin. The salt form may confer improved pharmacokinetic properties on the PGG as compared to the free form of the compound. The pharmaceutically acceptable salt form may also positively affect the pharmacodynamics of the compound with respect to its therapeutic activity in the body. An example of pharmacodynamics property that may be favorably affected is the manner in which the compound is transported across cell membranes, which in turn may directly and positively affect the adsorption, distribution, biotransformation and excretion of the compound.

The present invention also relates to a pharmaceutical composition comprising as an active ingredient PGG or a pharmaceutically acceptable salt thereof for use in treatment and/or prevention of a disease, e.g. of pulmonary hypertension; preferably the pulmonary hypertension is secondary pulmonary hypertension; more preferably the pulmonary hypertension is pulmonary hypertension secondary to left heart disease (LHD; WHO group II).

Such pharmaceutical composition may comprise, in addition to PGG or a pharmaceutically acceptable salt thereof, one or more pharmaceutically acceptable excipients. The term "excipient" is used herein to describe any ingredient other than the compound of the invention. The choice of excipient will to a large extent depend on the particular mode of administration. Excipients can e.g. be suitable carriers, retardants, boosters, prolonging substances, adjuvants, stabilizers, binders, emulsifiers, surface active agents, penetration enhancers suspending agents, disintegrants, buffers, salts, dilutents, solvents, dispersion media, fillers, lubricants, propellants, preservatives, flavours or mixtures thereof.

The pharmaceutical composition for use according to the present invention preferably comprises the pentagalloyl glucose combined with a delivery vehicle. The delivery vehicle is any compound or composition suitable for delivering the biologically active PGG or the pharmaceutically acceptable salt thereof to a location within the body, where the biological activity is needed or desired. Examples of suitable delivery vehicles comprise microparticle, nanoparticle, hydrogel, perivascular drug delivery vehicle, endovascular drug delivery vehicle, a stent or a combination thereof.

Perivascular delivery vehicle technologies suitable for use in the present invention are generally known to those of skill in the art, and, thus, need not be explained at length herein. For instance, exemplary known perivascular drug delivery technologies include those described by Chen, et al. (U.S. Patent Application Publication No. 2005/0079202) and Nathan (U.S. Patent Application Publication No. 2003/0228364). These exemplary perivascular delivery systems include a polymeric delivery vehicle that can be injected or directly placed, for instance via surgery, at a particular location so as to provide controlled release of the PGG compound encapsulated or otherwise loaded therein over a period of time.

Many endovascular delivery vehicles are likewise known in the art. For example, DiCarlo, et al. (U.S. Patent No. 6,929,626) describe an intraluminally placeable tubular device that can be located within the lumen of a blood vessel and coated or otherwise loaded with a drug, e.g., the PGG compounds described herein. The tubular member includes yarns interconnected in a pattern defining opposed interior and exterior textile surfaces. At least one of the textile surfaces is the body fluid-contacting luminal surface or the body lumen-contacting exterior surface. Wu, et al. (U.S. Patent No. 6,979,347) describe an apparatus and associated method for delivering a therapeutic substance such as the PGG compounds of the present invention, to a vascular lumen. Specifically, an implantable prosthesis, such as a stent, can be utilized that has grooves or trenches formed thereon. The grooves are formed on specific regions of the stent struts to increase the flexibility of the stent. The grooves also provide a location for carrying the PGG compound for delivery from the device following implantation. For example, the PGG compound, or a pharmaceutical composition thereof, can be deposited directly in to the grooves using conventional spray or modified dip techniques.

In another embodiment, the pharmaceutical composition of the invention can be administered by use of a hydrogel delivery vehicle. Hydrogels are herein defined to include polymeric matrices that can be highly hydrated while maintaining structural stability. Suitable hydrogel matrices can include un-crosslinked and crosslinked hydrogels. In addition, crosslinked hydrogel delivery vehicles of the invention can optionally include hydrolyzable portions, such that the matrix can be degradable when utilized in an aqueous environment, e.g., in vivo. For example, the delivery vehicle can include a cross-linked hydrogel including a hydrolyzable cross-linking agent, such as polylactic acid, and can be degradable in vivo. Hydrogel delivery vehicles of the present invention can include natural polymers such as glycosaminoglycans, polysaccharides, proteins, and the like, as well as synthetic polymers, as are generally known in the art. A nonlimiting list of hydrophilic polymeric materials that can be utilized in forming hydrogels of the present invention can include dextran, hyaluronic acid, chitin, heparin, collagen, elastin, keratin, albumin, polymers and copolymers of lactic acid, glycolic acid, carboxymethyl cellulose, polyacrylates, polymethacrylates, epoxides, silicones, polyols such as polypropylene glycol, polyvinyl alcohol and polyethylene glycol and their derivatives, alginates such as sodium alginate or crosslinked alginate gum, polycaprolactone, polyanhydride, pectin, gelatin, crosslinked proteins peptides and polysaccharides, and the like.

The delivery vehicles of the present invention can include a combination of one or more delivery vehicles. For instance, a hydrogel delivery vehicle can be combined with a patch, a stent, a perforated balloon, a vascular graft, or any other suitable device, for delivery of the disclosed agents to connective tissue.

In a preferred embodiment, the delivery vehicle comprises or consists of a microparticle or a nanoparticle. Particularly suitable micro- and nanoparticles are disclosed in US 2014/0017263 A1.

In general, any bulk biocompatible material capable of being formed to a useful size can be utilized in forming the micro- or nanoparticles of the delivery vehicles. In one embodiment, a polymeric particle can be utilized. For instance, particles formed from polystyrene, poly(lactic acid), polyketal, butadiene styrene, styrene-acrylic-vinyl terpolymer, poly(methyl methacrylate), poly(ethyl methacrylate), poly(alkyl cyanoacrylate), styrene-maleic anhydride copolymer, poly(vinyl acetate), poly(vinyl pyridine), poly(divinylbenzene), poly(butylene terephthalate), acrylonitrile, vinyl chlorideacrylates, poly(ethylene glycol), and the like, or an aldehyde, carboxyl, amino, hydroxyl, or hydrazide derivative thereof can be utilized.

Particles formed of biological polymers such as proteins can be used. For instance, particles formed of albumin, dextran, gelatin, chitosan, etc. can be utilized, preferably albumin is used for forming particles. Such particles can be preferred as they can be formed without the use of organic solvents according to known methods.

Other biocompatible materials as may be utilized in forming disclosed particles can include, without limitation, oxides such as silica, titania, zirconia, and the like, and noble metals such as gold, silver, platinum, palladium, and the like. In general, the materials will be biocompatible and nonimmunogenic.

The particles can be biodegradable. For instance, biodegradable polymeric particles formed from polysaccharide and/or poly(lactic acid) homopolymers and copolymers can be used. For example, particles formed of poly(lactic-co-glycolic acid) (PLGA) copolymers and derivatives thereof can be utilized.

In one embodiment, a poly(ethylene glycol) (PEG)/poly(lactic acid) (PLA) block copolymer can be utilized in forming the particles. A PEG-PLA block copolymer is an amphiphilic polymer with good stability in vivo. With good biocompatibility, the PEG hydrophilic component of the block copolymer can increase the solubility of insoluble compounds, prevent protein absorption on the particle surface, make the particles unrecognizable by the reticuloendothelial system as foreign bodies, and thereby provide particles that can have a characteristic of long circulation.

Selection of bulk nanoparticle material can be utilized to provide primary control of release rate of a biologically active compound from the loaded particle. For instance, selection of a biodegradable material can be utilized to increase the rate of compound release and provide a release mechanism that can be limited to a large extent by nanoparticle degradation rate and to a lesser extent by diffusion of the active compound from the bulk nanoparticle. Alternatively, materials can be utilized such that active compound release rate is limited by only diffusion (e.g., a nondegradable particle) or nanoparticle degradation rate (e.g., essentially no diffusion of the active compound through the particle due to small matrix mesh size).

As mentioned, the particles of the delivery agents can be microparticles or nanoparticles. By way of example, the size, i.e., the average diameter of formed nanoparticles can generally be less than about 500 nanometers, for instance less than about 200 nm, or less than about 100 nm. In one particular embodiment, the nanoparticles can be less than about 50 nm in size, for instance about 20 nm in average diameter. In one embodiment, nanoparticles can be formed having an average diameter of between about 50 nm and about 400 nm, or between about 100 nm and about 300 nm. In one embodiment, the nanoparticles can have an average diameter of about 200 nm.

Larger particles can alternatively be formed. For instance, in other embodiments, microparticles can be formed having a size of up to about 50 micrometers (µm). In general, the preferred size of the particles can depend upon the specific application, e.g., the specific method of delivery of the agents such as via surface application (as in a cream or lotion), via parenteral injection using the respiratory or digestive tract, etc., as well as the desired release rate of a treatment compound from the particles. For instance, in one embodiment, the particles can be of a size to prevent cellular uptake so as to remain in the extracellular matrix and available for interaction with damaged elastic fibers. Thus, the particles may be larger than about 100 nm in one embodiment, as smaller particles have been shown to exhibit higher cellular uptake. Particles can also be small enough so as to penetrate endothelium and penetrate basement membrane so as to contact the elastin fibers of the connective tissue. For instance, particles can be less than about 400 nm in average diameter in one embodiment so as to penetrate endothelium and basement membrane.

Generally, the particles are substantially spherical in shape, although other shapes including, but not limited to, plates, rods, bars, irregular shapes, etc., are suitable for use. As will be appreciated by those skilled in the art, the composition, shape, size, and/or density of the particles may vary widely.

Particles can be designed with a desirable surface charge so as to better target damaged elastin. For instance, positively charged nanoparticles have shown superior cellular uptake as compared to negatively charged particles. Thus, in one embodiment, particles can be developed with a negative surface charge to maintain the particles in the extracellular matrix and avoid cellular uptake.

Disclosed particles can be loaded with one or more biologically active compounds according to any suitable method. For instance, a precipitation method can be utilized to form the loaded particles in a one-step formation process. According to this method, a particle bulk material (e.g., a biocompatible polymer such as poly-(D,L-lactide-co-glycolide or a PGA/PLA copolymer) can be dissolved in a solvent to form a first solution. Suitable solvents can depend upon the specific materials involved. For example, organic solvents including acetone, tetrahydrofuran, dimethylsulfoxide, dimethylformamide, or acetonitrile and the like can be utilized. This first solution can undergo standard processing such as sonication, etc., so as to adequately solubilize the polymer. The first solution can then be added, generally dropwise, to a second solution. The second solution can be, e.g., an aqueous solution. Either spontaneously or following an emulsification method, for instance following sonication, particles can form that include the polymer bulk material.

According to a single-step formation process, a biologically active compound (e.g., pentagalloyl glucose (PGG)) can be included in either the first solution or the second solution. Upon formation of the particles, the biologically active compound, e.g. PGG, can be incorporated in the particles with the polymer bulk material.

Initial concentration of PGG within or on a particle can vary. For example, in one embodiment, loading concentration of biologically active compound like e.g. PGG in a particle can vary from about 4 wt. % to greater than about 40 wt. % by weight of the particle, with higher and lower concentrations possible depending upon specific compound, particle bulk material, and the like. For instance, in an embodiment in which a biologically active compound exhibits high solubility in the bulk particle material, a very high loading level can be attained, particularly when both materials are highly hydrophobic.

Precipitation methods can be useful, as such methods can provide monodisperse polymer particles loaded with a biologically active compound like e.g. PGG. Moreover, precipitation formation processes can be adjusted according to processing methods known to those skilled in the art to provide particles of a desired size and including a desired concentration of biologically active compound. For instance, modification of particle size can be obtained through modification of the concentration and/or type of surfactant included in the receiving solution according to known practices.

Formation processes can include two step processes in which particles are first formed followed by a second loading step in which PGG or more active agents are loaded into the formed particles. For instance, a method can include swelling a pre-formed, crosslinked polymeric particle in a solution that includes the biologically active compound so as to load the particle via a diffusion process. In another embodiment, loading method can include double emulsion polymerization, which enables loading of hydrophilic compounds into hydrophobic particles.

Methods of forming particles loaded with a biologically active compound like e.g. PGG are not limited to the precipitation method. Other micro- and nanoparticle formation processes as are known in the art as can be utilized in forming particles loaded with active compounds. For instance, supercritical fluid processing methods as disclosed by U.S. Pat. No. 7,754,243 to Sun can be utilized to form extremely small nanoparticles, e.g., less than about 20 nm having a very narrow size distribution and little or no particles that do not include the biologically active compound in the as-formed suspension.

Loaded particles can be formed so as to control the rate of release of active compound from a particle. Suitable control mechanisms are known to those of skill in the art. For instance, release rates can depend upon the relative concentration of active compound to bulk particle material, upon the molecular weight and degradation characteristics of the bulk nanoparticle material, upon the mesh size of a polymer particle matrix, upon the binding mechanism between the surface of a particle and an active compound, and so forth, as is known. In any of these cases, one of ordinary skill in the art is capable of engineering a system so to achieve desirable release rate. For instance, in the case of purely diffusion-limited release, such control can be achieved by variation of compound concentration within particles and/or particle size, particle polymer mesh size, and so forth. In the case of purely degradation-limited release, polymer monomer units, for instance glycolic acid content of a PLGA polymer, and/or molecular weight of particle bulk material, as well as particle size, can be adjusted to "fine tune" active compound release rate. For example, use of PLGA polymers with higher glycolic acid content and lower molecular weight can lead to an increased degradation rate of a particle formed with the polymer. Release rate of active compound from particles can be adjusted utilizing the above parameters so as to produce carriers capable of sustained release for periods varying from a few days to a few months, with the maximum release rates generally varying from a few hours to a few weeks.

According to another embodiment, release rate of an active compound can be controlled through binding, generally noncovalent binding, of the active compound to a ligand within the particle. Exemplary methods and materials of as can be utilized in one embodiment are described in U.S. Pat. No. 8,128,952 to Metters, et al., which is incorporated herein by reference. According to this method, a ligand can be selected that has an affinity for the biologically active compound to be delivered by the agent. For instance, a ligand can be selected according to a predetermined dissociation constant (K_{D}) describing this affinity and the ligand can be incorporated into the particle at a predetermined concentration level. The rate of release of the active compound from the particle that is established upon incorporation of the compound into the particle can then be controlled according to these particular parameters, i.e., K_{D} and the concentration of the ligand.

Biologically active compounds, like PGG, need not necessarily be incorporated within the bulk particle material. For example, in another embodiment, a biologically active compound can be bound to the surface of a particle. For example, a compound can be bound to the surface of a particle utilizing chemistry similar to that as is described in more detail below with regard to the binding of the targeting antibodies to the particles, e.g., via glutaraldehyde crosslinking.

Delivery vehicles can include additional materials on or within the particles, in addition to PGG or more active compounds that can treat elastic fiber degradation. Such materials can be active materials, providing direct benefit to the tissue in addition to the stabilization provided by the biologically active compound, or may be supporting materials, improving delivery, compatibility, or reactivity of other materials in the delivery agent. For example, in one embodiment, the delivery vehicle can include glutaraldehyde. Glutaraldehyde, when targeted to connective tissue, can form covalent cross-links between free amines in proteins in order to further stabilize the tissue.

In addition to the particle and PGG (and optionally further biologically active compounds), the delivery vehicle preferably includes an anchoring agent that binds at or near degraded elastic fibers so as to provide the PGG compound at the targeted site. Preferably, the anchoring agent binds specifically to a structure associated with a pulmonary blood vessel; more preferably to a structure of a cell of a pulmonary blood vessel or a component of the extracellular matrix of a pulmonary blood vessel. Preferably, the loaded particle can be coated with an anchoring agent that can specifically bind elastin, preferably human elastin, as degraded elastic fibers will include elastin exposed due to degradation of the microfiber scaffolding. Accordingly, in one embodiment, a delivery vehicle can include at a surface an antibody or a fragment thereof that is specific for elastin for targeting the vehicle to degraded elastic fibers and providing the PGG compound of the delivery vehicle to the damaged elastic fibers at the anchoring site.

The anchoring agent bound to a surface of a particle can be a polypeptide, e.g., either a complete protein or a fragment thereof, that can recognize and bind receptors at the targeted site. This is not a requirement of the disclosed anchoring agents, however, and in another embodiment, an anchoring mechanism can utilize a non-proteinaceous anchoring agent, for instance a polysaccharide that can bind a particle to a targeted location, e.g., elastin exposed due to degradation of the elastic fiber.

Anchoring agents can be natural or synthetic agents. The anchoring agent can include polyclonal or monoclonal antibodies as desired. Antibodies can be raised according to known methods. Preferably, the anchoring agent is an antibody or an antigen binding fragment thereof.

As used herein, the term "antibody" refers to single chain, two-chain, and multi-chain proteins and glycoproteins that belong to the classes of polyclonal, monoclonal, chimeric and human or humanized immunoglobulin proteins. The term "antibody" also includes synthetic and genetically engineered variants thereof.

As used herein, the term "antibody fragment" or "antigen binding fragment" of an antibody refers to a Fab fragment, a Fab' fragment, a F(ab')2 fragment, a F(ab')3 fragment, a Fd fragment, a Fd' fragment, a Fv fragment, a scFv, a bivalent scFv, a diabody, a linear antibody, single chain antibodies, functional heavy chain antibodies (nanobodies), as well as any portion of an antibody having specificity toward at least one desired epitope, that competes with the intact antibody for specific binding (e.g., an isolated portion of a complementarity determining region having sufficient framework sequences so as to bind specifically to an epitope). Antigen binding fragments can be produced by recombinant techniques, or by enzymatic or chemical cleavage of an intact antibody.

As used herein, the term "human antibody" refers to an antibody that possesses a sequence that is derived from a human germ-line immunoglobulin sequence, such as antibodies derived from transgenic mice having human immunoglobulin genes (e.g., XENOMOUSE ^{™}genetically engineered mice (Abgenix)), human phage display libraries, in cows (milk) or human B cells.

As used herein, the term "humanized antibody" refers to an antibody that is derived from a nonhuman antibody (e.g., murine) that retains or substantially retains the antigen-binding properties of the parent antibody but is less immunogenic in humans. Humanized as used herein is intended to include deimmunized antibodies.

The term "modified" or "recombinant" antibody, as used herein, refers to antibodies that are prepared, expressed, created or isolated by recombinant means, such as antibodies expressed using a recombinant expression vector transfected into a host cell, antibodies isolated from a recombinant, combinatorial antibody library, antibodies isolated from an animal (e.g., a mouse) that is transgenic for human immunoglobulin genes or antibodies prepared, expressed, created or isolated by any other means that involves splicing of human immunoglobulin gene sequences to other DNA sequences. Such modified antibodies include humanized, CDR grafted, chimeric, *in vitro* generated (e.g., by phage display) antibodies, and may optionally include variable or constant regions derived from human germline immunoglobulin sequences or human immunoglobulin genes or antibodies which have been prepared, expressed, created or isolated by any means that involves splicing of human immunoglobulin gene sequences to alternative immunoglobulin sequences.

The term "monospecific antibody" refers to an antibody that displays a single binding specificity and affinity for a particular target, e.g., epitope. This term includes a "monoclonal antibody" or "monoclonal antibody composition," which as used herein refer to a preparation of antibodies or fragments thereof of single molecular composition.

The term "bispecific antibody" or "bifunctional antibody" refers to an antibody that displays dual binding specificity for two epitopes, where each binding site differs and recognizes a different epitope.

In a preferred embodiment, the antibody or the antigen binding fragment thereof to be used as anchoring agent specifically binds to elastin, preferably to human elastin.

Following formation, the anchoring agent can be further processed so as to facilitate conjugation with the particle of the delivery vehicle. For example, following initial formation of the anchoring agent, e.g., an antibody, the antibody can be further processed so as to be more readily bound to the particle of the delivery agent. For instance, antibody can be reacted with a thiolation compound such as Traut's Reagent (2-iminothiolane) to produce a thiolated antibody.

The antibody can be conjugated with the particle according to any suitable process. For example, a particle can include surface reactive groups to facilitate conjugation of the particle with an anchoring agent, e.g., an antibody. Surface reactive groups can include, without limitation, aldehyde, carboxyl, amino, hydroxyl, and the like. Surface reactive groups can either exist on the particle surface as formed or can be added to the surface following formation, for instance via oxidation, amination, etc., of the formed particle, as is generally known in the art. The antibody can then be conjugated with the particle, for instance through reaction with maleimide in the illustrated embodiment in which the antibody is a thiolated antibody as described above.

Anchoring agents can be attached to a particle via either nonspecific adsorption or a covalent bond. Preferred attachment methods can generally depend upon the desired application of the formed conjugates. For instance, in those embodiments in which a system is designed to function in vivo, the particles can be expected to encounter multiple collisions with various biological agents and tissues. Accordingly, covalent binding can be preferred in such an embodiment, to better ensure that the anchoring agents will not be dislodged through collision of the particles with other materials.

The specific chemistry utilized to bind the anchoring agent (and optionally the biologically active compound like e.g. PGG) to the particle surface is not particularly limited. For example, in one embodiment, a proteinaceous anchoring agent can be bound to a chloromethylated particle according to a nucleophilic substitution reaction between a protein amine group and the alkyl chloride of the particle. In another embodiment, soluble carbodiimide (EDC) and glutaraldehyde chemistry can be used to achieve covalent binding of amine groups of a proteinaceous agent to carboxylated and aminated particles, respectively. According to yet another embodiment, a proteinaceous agent can be bound to a particle through initial covalent attachment of a streptavidin monolayer to a particle followed by controllable attachment of desired amounts of biotinylated proteins. The presence of a streptavidin monolayer can also eliminate potential problems related to direct interaction of functional proteins with particles in the environment in which the particles will be utilized. According to yet another embodiment, a proteinaceous anchoring agent can be covalently attached to a particle using a crosslinking agent, for instance a phenylazide crosslinking agent such as sulfo-HSAB (N-Hydroxysulfosuccinimidyl-4-azidobenoate) a photoreactive reagent available from Pierce, Inc. that can crosslink amine groups of the proteinaceous anchoring agent and C—H or C—C bonds of a polymeric particle.

In one embodiment, a molecular spacer, for instance a hydrophilic spacer, can be utilized to tether an anchoring agent to a particle. Utilization of a spacer can prevent interaction of covalently bound anchoring agents, e.g., proteins, with the particle surface and thus prevent structural changes of the protein that can lead to partial or complete loss of functionality of the protein. Spacers can include long (e.g., weight average molecular weight between about 2,000 and about 20,000 Da) hydrophilic polymers so as to minimize interaction of the bound proteins with the surface of the particle. Hydrophilic spacers can include, without limitation, poly(ethylene glycol), polyvinyl alcohol, polysaccharides, and so forth.

In an exemplary method of attachment of a proteinaceous anchoring agent to a particle through a poly(ethylene glycol) (PEG) spacer, the PEG spacer and the particle can include or be processed to include functionality so as to facilitate binding to one another. For example, the PEG spacer can include aldehyde functionality and can bind to the aminated particle through covalent reaction between the aldehyde group of the spacer and the amine group of the particle. The thiolated antibody can then be attached to the spacer according to a simple process including mixing of a solution including the thiolated antibody with an aqueous suspension of particles in the presence of maleimide so as to form the delivery agent.

At the final stage of conjugation, a particle can be blocked, for instance, with a surfactant, such as Tween^{®} 20, Pluronic^{®}, or dextrane that can be adsorbed on the particle to block any hydrophobic surface exposed to the solution as well as to displace any noncovalently bound agents. Low concentrations of such materials generally do not interfere with the activity of agents such as water-soluble enzymes. The presence of a surfactant can reduce undesirable protein-particle interactions and prevent particle aggregation. It can also prevent nonselective "fouling" of the surface of a particle with other proteins in the environment in which the material is utilized that could potentially deactivate a system.

As previously mentioned, surface sites of a particle to which an anchoring agent can be bound can vary. For instance, in one embodiment, carboxyl-modified particles can be utilized. For example, carboxyl-modified PLA-based particles can be utilized. According to one such embodiment, an NH₂-PEG-COOH spacer can be bound to a particle via the amine group using carbodiimide chemistry according to known methodology. An anchoring agent can then be likewise coupled to the carboxyl group of the spacer using carbodiimide chemistry. The surface of the particle can then be blocked with a suitable agent (e.g., Tween^{®} 20, Pluronic^{®}, dextran, and the like), as described above.

Beneficially, it is possible to precisely engineer a particle to exhibit anchoring properties for a desired application. For example, the binding capacity and length of time a particle can remain bound to the targeted tissue, e.g., damaged vasculature, can be engineered by altering particle size, type of the anchoring agent, and/or anchoring agent concentration on the particle surface.

According to one embodiment, a single anchoring agent can bind to more than one particle. For instance, in those embodiments in which an anchoring agent can be bound to a particle via amine groups of the protein, and as protein molecules have more than one amine group, a single protein molecule can potentially bind to more than one particle. This may result in the formation of dimers and larger aggregates of particles. While formation of large aggregates may be preferred in some embodiments, for instance in some in vitro assay applications or in an in vivo topical application embodiment in other applications it can be preferable to minimize aggregation. Accordingly, in one embodiment, a lower particle concentration and/or a higher concentration of surfactant, as well as variation in surfactants, can be utilized during a formation process in order to minimize aggregation of particles.

In accordance with the present invention, PGG or a pharmaceutically acceptable salt thereof or the pharmaceutical composition of the invention is used in treatment and/or prevention of a disease, e.g. of pulmonary hypertension; preferably the pulmonary hypertension is secondary pulmonary hypertension; more preferably the pulmonary hypertension is pulmonary hypertension secondary to left heart disease (LHD; WHO group II).

According to WHO classification, pulmonary hypertension (PH) is divided into 5 separate groups, wherein Group I (pulmonary arterial hypertension) is further subdivided into Group I' and Group I" classes.

The most recent WHO classification system can be summarized as follows:
WHO Group I - pulmonary arterial hypertension (PAH);
WHO Group I' - pulmonary veno-occlusive disease (PVOD), pulmonary capillary hemangiomatosis (PCH);
WHO Group I" - persistent pulmonary hypertension of the newborn;
WHO Group II - pulmonary hypertension secondary to left heart disease;
WHO Group III - pulmonary hypertension secondary to lung disease like e.g. chronic hypoxia, chronic obstructive pulmonary disease (COPD), interstitial lung disease, mixed restrictive and obstructive pattern pulmonary diseases, sleep-disordered breathing, alveolar hypoventilation disorders, chronic exposure to high altitude, and/or developmental abnormalities;
WHO Group IV - chronic arterial obstruction;
WHO Group V - pulmonary hypertension with unclear or multifactorial mechanisms.

Preferably, PGG or a pharmaceutically acceptable salt thereof or the pharmaceutical composition of the invention are used in treatment and/or prevention of secondary pulmonary hypertension; more preferably of pulmonary hypertension secondary to left heart disease (LHD; WHO group II).

As used herein, the term "treating" encompasses reversing, alleviating or inhibiting the progress of a disease, disorder or condition, or improvement of one or more symptoms of such disease, disorder or condition, to which such term applies. As used herein, "treating" may also refer to decreasing the probability or incidence of the occurrence of a disease, disorder or condition in a mammal as compared to an untreated control population, or as compared to the same mammal prior to treatment. For example, as used herein, "treating" may refer to preventing a disease, disorder or condition, and may include delaying or preventing the onset of a disease, disorder or condition, or delaying or preventing the symptoms associated with a disease, disorder or condition. As used herein, "treating" may also refer to reducing the severity of a disease, disorder or condition or symptoms associated with such disease, disorder or condition prior to a mammal's affliction with the disease, disorder or condition. Such prevention or reduction of the severity of a disease, disorder or condition prior to affliction relates to the administration of the composition of the present invention, as described herein, to a subject that is not at the time of administration afflicted with the disease, disorder or condition. As used herein the term "treating" may also refer to preventing the recurrence of a disease, disorder or condition or of one or more symptoms associated with such disease, disorder or condition. The terms "treatment" and "therapeutically," as used herein, refer to the act of treating, as "treating" is defined above.

For the purpose of the present invention any reference to a method for treatment comprising the administration of a compound or to a use of a compound in a method of manufacture of a medicament for treatment of a disease is to be understood as a reference to said compound for use in such a method.

The present invention also relates to PGG or a pharmaceutically acceptable salt thereof or a pharmaceutical composition for use in manufacture of a medicament for treatment and/or prevention of pulmonary hypertension. Preferably, the pulmonary hypertension is secondary pulmonary hypertension; more preferably the pulmonary hypertension is pulmonary hypertension secondary to left heart disease (LHD; WHO group II).

The present invention also relates to a method of treatment of pulmonary hypertension, wherein a patient in need of such therapy is administered a therapeutically effective dose of PGG or a pharmaceutically acceptable salt thereof or a pharmaceutical composition comprising the same. Preferably, the pulmonary hypertension is secondary pulmonary hypertension; more preferably the pulmonary hypertension is pulmonary hypertension secondary to left heart disease (LHD; WHO group II).

The PGG or the pharmaceutically acceptable salt thereof of the pharmaceutical composition of the invention are administered preferably at an effective dose. An "effective dose" is the dose of PGG that upon administration to a patient yields a measurable therapeutic effect with regard to the disease of interest. In the present invention an effective dose is the dose of PGG that upon administration to a patient yields a therapeutic effect with regard to at least one disease related symptom in a patient or patients suffering from said disease. Preferably, PGG is administered at a dose of not more than 500 mg/kg/d. In particular, PGG can be administered at a dose of 1 mg/kg/d to 400 mg/kg/d, preferably of 20 mg/kg/d to 150 mg/kg/d. In any event, the physician or the skilled person will be able to determine the actual dose which will be suitable for an individual patient, which is likely to vary with the age, weight, sex, and concomitant illnesses such as renal or hepatic dysfunction and response of the particular patient to be treated. The above-mentioned dosages are exemplary of the average case. There can, of course, be individual instances where higher or lower dosage ranges are appropriate, and such are within the scope of the invention.

The PGG or pharmaceutically acceptable salt thereof or the pharmaceutical composition for use of the present invention are preferably administered orally, intravenously, subcutaneously, bucally, rectally, dermally, nasally, tracheally, bronchially or by any other parenteral route or via inhalation in a pharmaceutically acceptable dosage form. The pharmaceutical composition for use according to the invention is configured for e.g. topical or systemic administration, preferably for intravascular, intravenous, intra-arterial, intracardial, intra-pulmonal and/or nasal administration.

The PGG or pharmaceutically acceptable salt thereof or the pharmaceutical composition for use of the present invention may be administered orally. Oral administration may involve swallowing, so that the compound enters the gastrointestinal tract, or buccal or sublingual administration may be employed by which the compound enters the blood stream directly from the mouth.

Formulations suitable for oral administration include: solid formulations such as tablets; capsules containing particulates, liquids, or powders; lozenges (including liquid-filled); and chews; multi- and nano-particulates; gels; solid solutions; liposomes; films, ovules, sprays and liquid formulations. Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

For tablet dosage forms, depending on dose, the compound may make up from 1 weight % to 80 weight % of the dosage form, more typically from 5 weight % to 60 weight % of the dosage form. In addition to the compound, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pre-gelatinized starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80% compound, from about 10 weight % to about 90 weight % binder, from about 0 weight % to about 85 weight % diluent, from about 2 weight % to about 10 weight % disintegrant, and from about 0.25 weight % to about 10 weight % lubricant.

The PGG or pharmaceutically acceptable salt thereof or the pharmaceutical composition for use of the present invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water. The preparation of parenteral formulations under sterile conditions, for example, by lyophilization, may readily be accomplished using standard pharmaceutical techniques well known to those skilled in the art.

The PGG or pharmaceutically acceptable salt thereof or the pharmaceutical composition for use of the present invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol.

The PGG or pharmaceutically acceptable salt thereof or the pharmaceutical composition for use of the present invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electro-hydrodynamics to produce a fine mist), or nebulizer, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin. The pressurized container, pump, spray, atomizer, or nebulizer contains a solution or suspension of the 4,5-diarylimidazole derivatives of the invention comprising, for example, ethanol, aqueous ethanol, or a suitable alternative agent for dispersing, solubilizing, or extending release of the active, a propellant(s) as solvent and an optional surfactant, such as sorbitan trioleate, oleic acid, or an oligolactic acid.

The use of PGG or pharmaceutically acceptable salt thereof or the pharmaceutical composition in the treatment of pulmonary hypertension, preferably of pulmonary hypertension secondary to left heart disease, may have the advantage that such compound may be more efficacious than, be less toxic than, be longer acting than, be more potent than, produce fewer side effects than, be more easily absorbable than, have better pharmacokinetic profile (e.g. higher oral bioavailability and/or lower clearance) than, and/or have other useful pharmacological, physical, or chemical properties over compounds known in the prior art for treatment of said diseases.

### FIGURES:

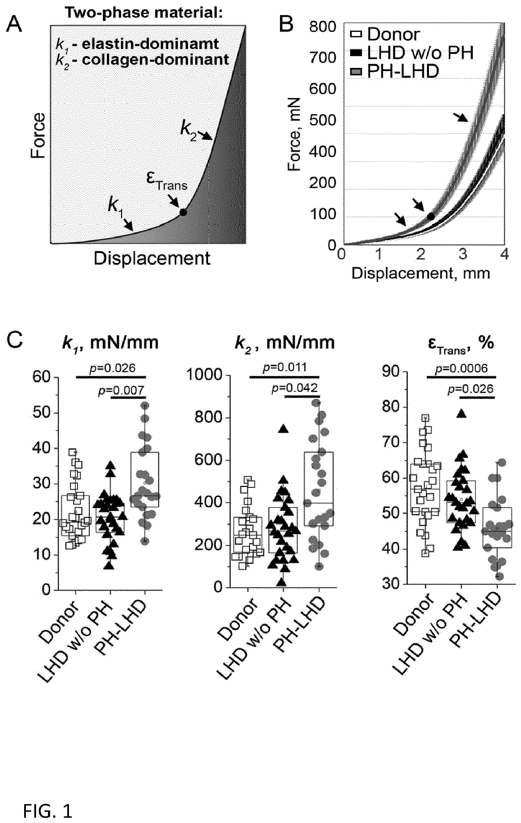

**Figure 1****. Conduit pulmonary arteries are stiffened in PH-LHD**
   **A,** Schematic illustration depicting the typical force-displacement curve of a conduit artery undergoing a uniaxial tensile test. *k₁*, stiffness of elastin-dominamnt material; *k*₂, stiffness of collagen-dominant material; εTrans, theoretical strain value at which the elastin-dominant material property shifts to a collagen-dominant material property. **B**, Group data show force-displacement curves (mean±SEM) for PAs from donors (n=26), LHD w/o PH patients (n=28), and PH-LHD patients (n=22). **C**, Box-and-whisker plots show *k₁*, *k*₂, and εTrans in PAs from donors, LHD w/o PH patients, and PH-LHD patients respectively.
   *Statistics:* Kruskal-Wallis one-way ANOVA on ranks followed by pairwise multiple comparisons (Dunn's test).
**Figure 2****. Pulmonary artery biomechanical competences correlate with pulmonary arterial pressure**
   Scatter diagrams show the relationship between PAPm and parameters of pulmonary arterial biomechanics, *k₁*, *k*₂, and εTrans.
   *Statistics:* Spearman's coefficient of correlation Rho (r) and corresponding p-values are presented in each panel.
**Figure 3****. Progressive remodeling of elastic and collagen fibers in PH-LHD**
   **A**, Representative images of EVG-stained PA medial walls from a donor, an LHD w/o PH patient and a PH-LHD patient. Left panels show elastin-positive area only, right panels show collagen only. Notably, the hematoxylin stain with EVG stain also stains cell nuclei, which are hence included in the elastin stain signal on the left panel. **B,** Box-and-whisker plots show area of elastic and collagen particles in LHD w/o PH and PH-LHD samples normalizes to the image area and to the donor control. **C**, Box-and-whisker plots showing the collagen-to-elastin ratio in PA samples of donors, LHD w/o PH patients, and PH-LHD patients.
   *Statistics:* Kruskal-Wallis one-way ANOVA on ranks followed by pairwise multiple comparisons (Dunn's test).

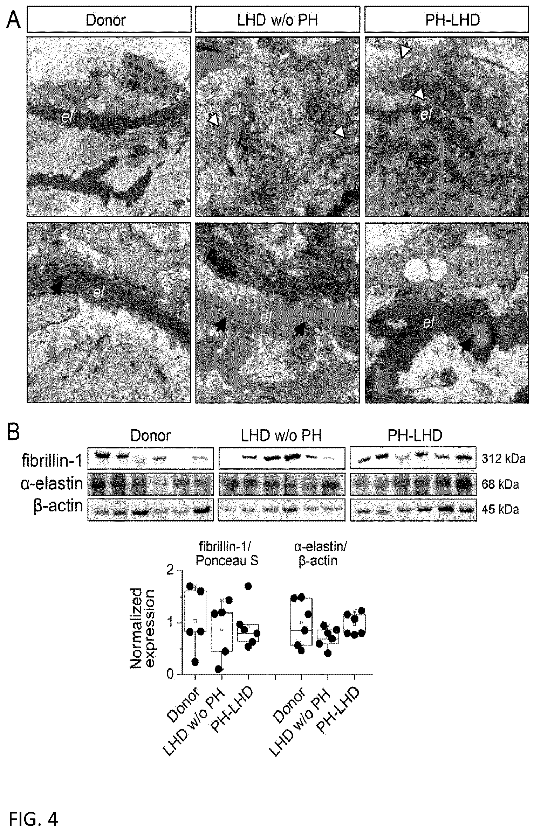

**Figure 4****. Ultrastructure and composition of elastic fibers**
   **A,** TEM images show the ultrastructure of the PA media in samples from a donor, an LHD w/o PH patient and a PH-LHD patient. *el,* elastic fibers; arrows in white - fragmented elastic fibers, black - elastin core within elastic fibers. **B**, Western blots and quantitative densitometric data (box-and-whisker plot) show the expression of the elastic fiber components α-elastin and fibrillin-1 in the PAs of donor (n=6), LHD w/o PH (n=6) or PH-LHD (n=6) patients.
   *Statistics:* Kruskal-Wallis one-way ANOVA on ranks followed by pairwise multiple comparisons (Dunn's test).

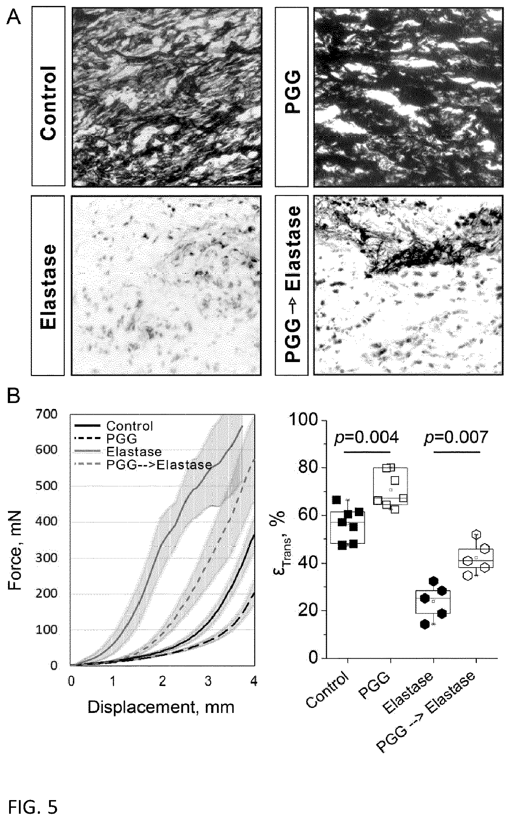

**Figure 5****. Elastin stabilization with PGG protects against elastolysis and improves pulmonary artery biomechanics**
   **A**, Representative images of a donor PA wall stained with EVG stain show elastin (violet) in arterial media for control (DMSO), PGG-treated, elastase-treated, and PGG→elastase-treated samples. B, Group data show force-displacement curves (mean±SEM) for donor PAs after 24 h of ex *vivo* culturing in the presence or absence of elastase or PGG, respectively. Box-and-whisker plot showing εTrans calculated from force-displacement curves.
   *Statistics:* c, Mann-Whitney U test.

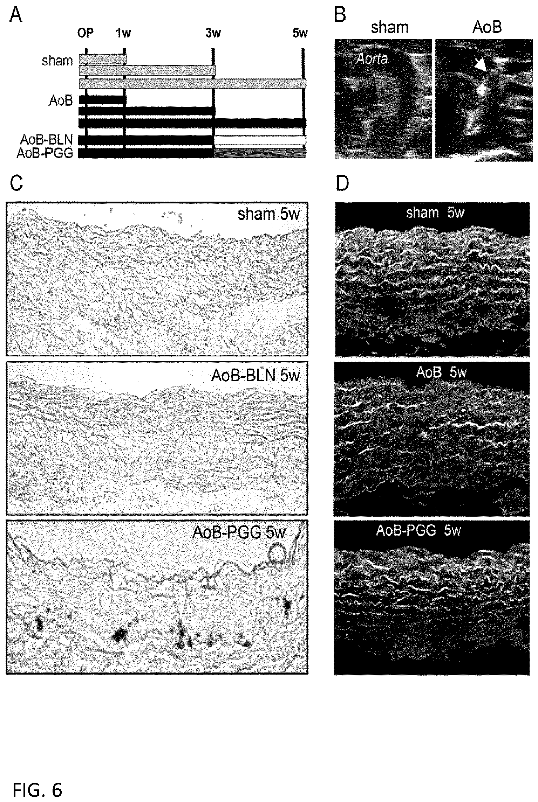

**Figure 6****. Targeted delivery of PGG to pulmonary artery sites PH in a rat model of PH-LHD**
   **A**, Schematic depiction of the experimental protocol. Animals underwent sham surgery for AoB with or without PGG treatment and were analyzed after 1 week (1w; sham, AoB), 3 weeks (3w; sham, AoB), or 5 weeks (5w; sham, AoB, AoB-BLN, AoB-PGG) (n=8-12 animals per group). OP, operation (AoB or sham); white and dark grey bars indicate the time frame of EL-BLN-NP or EL-PGG-NP treatment. **B**, Representative echocardiographic images show clip placement (yellow arrow) on the ascending aorta in AoB animals compared to sham rats. **C**, Representative bright-field microscopy images show the PA from a sham rat, an AoB rat, and an AoB-PGG rat 5 weeks post AoB. PGG was detected by FeCl₃ staining in the PAs of AoB-PGG rats. **D**, Representative images show elastic fibers as visualized by autofluorescence in the PAs of sham, AoB, and AoB-PGG rats 5 weeks after surgery.

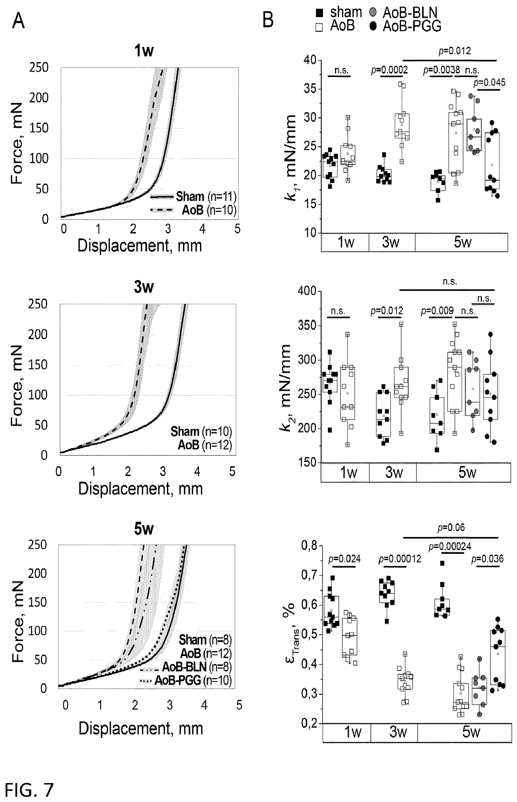

**Figure 7****. Targeted delivery of PGG attenuates PA stiffening in a rat model of PH-LHD**
   **A,** Group data show force-displacement curves (mean±SEM) for the PAs of AoB and sham animals at 1, 3, and 5 weeks after surgery and in AoB-BLN and AoB-PGG rats after 5 weeks. **B**, Box-and-whisker plots show *k₁*, *k₂* and εTrans for PAs from sham, AoB, AoB-BLN and AoB-PGG rats.
   *Statistics:* Kruskal-Wallis one-way ANOVA on ranks followed by pairwise multiple comparisons (Dunn's test).

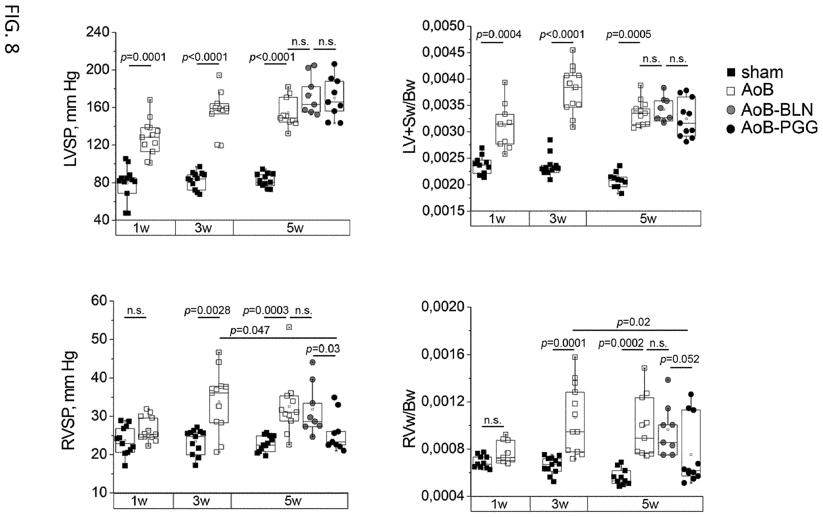

**Figure 8****. Targeted delivery of PGG attenuates pulmonary hypertension in a rat model of PH-LHD**
   Box-and-whisker plots show left (LVSP) and right (RVSP) ventricular systolic pressures assessed by cardiac catheterization and left (LV+Sw/Bw) and right (RVw/Bw) ventricular weights normalized to bw.
   *Statistics:* Kruskal-Wallis one-way ANOVA on ranks followed by pairwise multiple comparisons (Dunn's test).

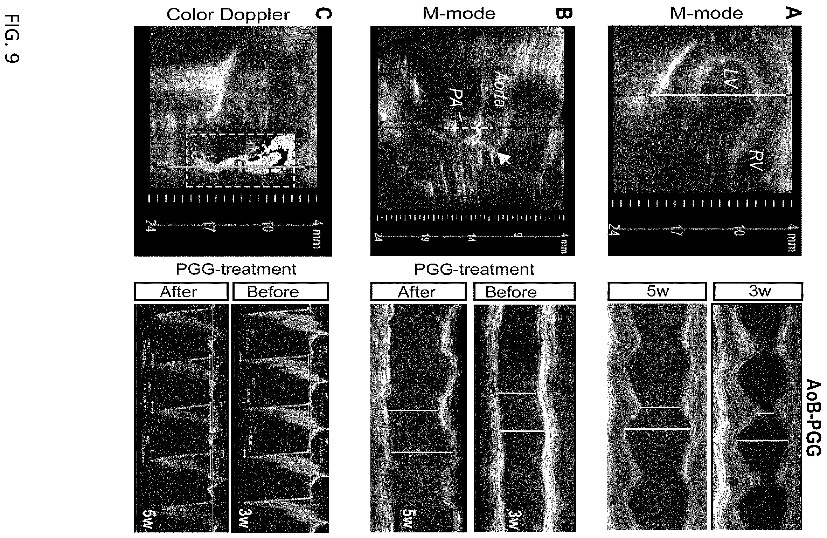

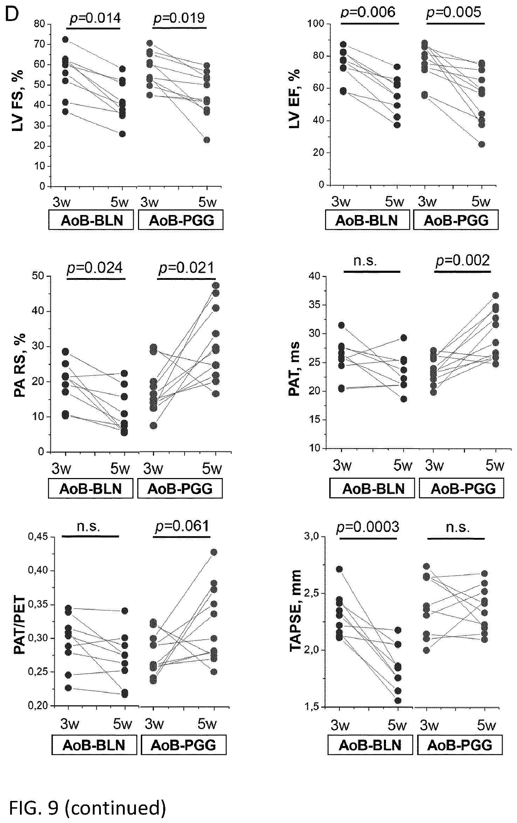

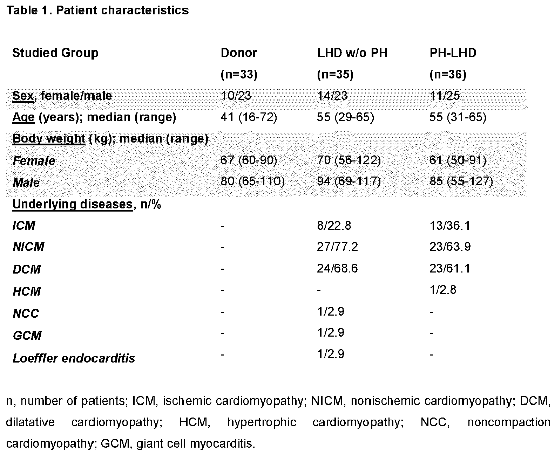

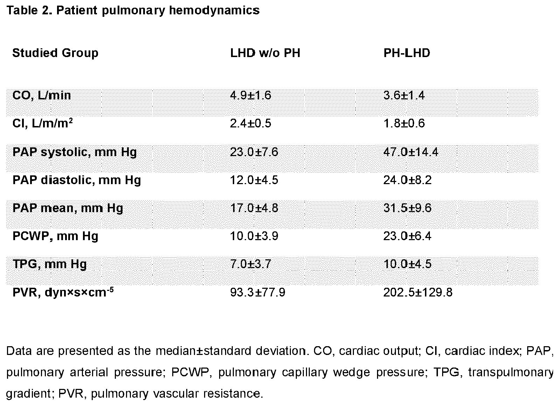

**Figure 9****. Treatment with PGG improves pulmonary arterial biomechanics and hemodynamics in a rat model of PH-LHD**
   A, Representative M-mode images acquired by transthoracic echocardiography display dimensions of the LV walls and LV cavity in an AoB-PGG rat at 3 weeks and 5 weeks after surgery. In comparison to 3 weeks, LV shortening was notably reduced at 5 weeks. B, M-mode images show PA distensibility in an AoB-PGG rat before (3w) and after (5w) PGG treatment. Arrow points to the clip on the aorta. C, Representative images show pulmonary blood flow as detected by pulse-wave and color Doppler echocardiography and the analysis of PAT and pulmonary ejection time (PET) parameters in an AoB-PGG rat before (3w) and after (5w) PGG treatment. D, Line graphs show longitudinal changes in left ventricular fractional shortening (LV FS), left ventricular ejection fraction (LV EF), pulmonary artery radial strain (PA RS), pulmonary acceleration time (PAT), PAT/PET and tricuspid annular plane systolic excursion (TAPSE) in AoB-BLN and AoB-PGG rats before (3w) and after (5w) treatment with either vehicle or PGG. *Statistics:* Wilcoxon matched-pairs signed rank test.

### EXAMPLES:

### Materials and methods:

### Collection of human PA samples and clinical data analyses

Human tissue samples were collected following approval by the Ethics Committee of the Charité-University Medicine Berlin (EA4/035/18) and with the informed consent of the patients. Specimens from the pulmonary trunk (hereafter referred to as PA samples) were collected during orthotopic heart transplantation from donors (healthy-heart control group, n=33), patients with LHD without PH (LHD w/o PH group, n=35), and patients with PH due to LHD (PH-LHD group, n=36), when the length of the PA was adjusted before anastomosis. The diagnosis of PH was verified by data from right heart catheterization (RHC) obtained within 6 months prior to transplantation using a cut-off value of a mean PAP of 25 mm Hg according to current guidelines.

Demographic data for age and sex did not differ significantly between healthy-heart donors and LHD w/o PH and PH-LHD patients and are reported in Table 1. Underlying diseases comprised ischemic cardiomyopathy and nonischemic cardiomyopathy for patients with LHD w/o PH and PH-LHD (Table 1). Pulmonary hemodynamics for LHD w/o PH and PH-LHD patients are summarized in Table 2.

### Biomechanical testing of human PA samples

**Uniaxial tensile test.** Following harvesting, human PA samples were kept in normal saline on ice and assessed for biomechanics within 2-4 h. Samples were checked for holes or tears, the presence of which resulted in exclusion from biomechanical testing. Loose connective and adipose tissue were carefully removed, and sample dimensions (length and width) were measured using a digital caliper. The circumferential tensile properties of PAs were assessed using a MyoDynamics Muscle Strip Myograph System (840DM, Danish Myo Technology, Hinnerup, Denmark) with controlled temperature (37°C) and aeration. To this end, circumferential rectangular sections (2x8 mm) were excised from the pulmonary trunk and mounted at 5 mm length. Arterial tissue was preconditioned by 5 extension-relaxation cycles at a low force of 5-10 mN to avoid damaging the elastin material. After sample equilibration at a baseline force of 1 mN, an automated displacement (ΔL) of 4 mm was applied at a rate of 0.5 mm/s. In each tested sample, a full strain (ε) of 80% was achieved. The generated force (F) was recorded in real time by a data acquisition system (PowerLab, ADInstruments) and displayed as a force-displacement curve. Between 2 and 4 specimens of each PA were tested, and the results for these technical replicates were averaged. Force-displacement curves show the typical characteristics of an arterial "two-component" material reflecting the tensile properties of elastin and collagen (Figure 1A). From force-displacement curves, the following parameters were derived: stiffness of elastin (*k₁*)- and collagen (*k₂*)-enriched arterial material (calculated as the slopes of the toe- and linear regions, respectively), with stiffness *(k,* mN/mm) reflecting the resistance to deformation (ΔF/ΔL) generated by the respective material, and the strain at which the transition of load-bearing elements from elastin to collagen occurred (εTrans), defined based on force-displacement curve characteristics as the strain applied when F reached 100 mN.

**Effects of PGG on PA biomechanics**. The effect of PGG on PA biomechanical properties was assessed by ex vivo culture of PA specimens followed by uniaxial tensile testing. Freshly isolated PAs were handled under sterile conditions. One piece of the artery was excised and directly underwent uniaxial tensile testing to record PA biomechanics at baseline. The rest of the PA was prepared as 2x8-mm strips and placed in a 12-well plate for ex vivo culture in serum-free Dulbecco's modified Eagle's medium (DMEM, Gibco) supplied with penicillin-streptomycin (Thermo Fisher) at 37°C with controlled humidity at 21% O₂ and 5% CO₂. PGG (penta-O-galloyl-β-D-glucose hydrate, Sigma-Aldrich) was dissolved in dimethyl sulfoxide (DMSO, Sigma-Aldrich) as a stock solution and added to the medium at a final concentration of 0.1%. Similar concentrations of PGG have previously been shown to have minimal cytotoxicity. Type IV elastase from porcine pancreas (E0258, Sigma-Aldrich) was prepared in Dulbecco's phosphate-buffered saline (DPBS, Gibco) as a stock solution, and 1 U was added to the medium based on the results from previous studies in porcine aortas demonstrating effective digestion of the majority of elastic fibers over 24 h. The following four treatment conditions were tested: control, elastase only, PGG only and PGG prior to elastase. PGG treatment was performed for 1 h at 37°C prior to culturing PAs for 24 h in the presence or absence of elastase. Control samples were incubated with the corresponding concentrations of DMSO.

### Rat model of PH secondary to LHD

All animal procedures were approved by the local governmental animal care and use committee (Landesamt für Gesundheit und Soziales (LaGeSO), Berlin) under protocol number G0030/18. All experiments were performed in accordance with the ARRIVE guidelines and the "Guide for the Care and Use of Laboratory Animals" (Institute of Laboratory Animal Resources, 7th edition 1996).

**Surgical procedure.** Congestive heart failure was surgically induced in juvenile Sprague-Dawley rats (approx. 100 g body weight (bw)) from Janvier Labs by supracoronary AoB as previously described. In brief, rats were anesthetized by intraperitoneal injection of ketamine (87 mg/kg bw) and xylazine (13 mg/kg bw), and adequate depth of anesthesia was checked regularly by the toe pinch test. In rats in the AoB group, a titanium clip of 0.8-mm inner diameter was placed on the ascending aorta. Perioperatively, rats were mechanically ventilated with room air at tidal volumes of 6 mL/kg bw via tracheotomy as previously described. Sham animals underwent all anesthetic and surgical procedures except for clip implantation and served as controls. Animals received moisturizing ointment for their eyes during anesthesia, as well as pre- and postoperative analgesia (carprofen, 5 mg/kg bw intraperitoneally daily for 1 week) and postoperative antibiotics (amoxicillin, 500 mg/L in drinking water). At 1, 3, or 5 weeks after surgery, endpoint measurements were performed, including invasive hemodynamic monitoring, the measurement of heart weight, postmortem control of clip placement, the testing of PA biomechanical properties and PA histological analyses as specified below.

**Preparation of PGG loaded bovine serum albumin (PGG-NPs) and blank nanoparticles (BLN-NPs), and antibody conjugation**. PGG-loaded bovine serum albumin (BSA) NPs (PGG-NPs) and blank BSA NPs (BLN-NPs) were prepared by previously described methods. Briefly, 250 mg of BSA (Seracare, Milford, MA) was dissolved in 4 mL of DI water, PGG solution (125 mg PGG (Ajinomoto Omnichem) dissolved in 400 µL dimethyl sulfoxide) was added while stirring, followed by 37 µL of 8% glutaraldehyde for crosslinking at room temperature. After 1 h of stirring, the NPs mixture was added slowly to 24 mL of ethanol (Sigma, St. Louis, MO) under continuous sonication (Omni Ruptor 400 Ultrasonic Homogenizer, Omni International Inc, Kennesaw, GA) for 30 min. The pellet of PGG-NPs was obtained after centrifugation at 6,000 rpm for 10 min. Blank (BLN) nanoparticles were prepared by omitting PGG addition.

PGG-NPs and BLN-NPs thus prepared were next conjugated with degraded elastin targeting rabbit anti-rat elastin polyclonal antibody (in house developed at Clemson University) for overnight as described previously. Briefly, 10 mg of NPs or PGG-loaded or blank NPs were PEGylated with 2.5 mg α-maleimide-ω-N-hydroxysuccinimide ester poly (ethylene glycol) (mPEG-NHS, M.W. 2000, Nanocs, NY, U.S.A.) at room temperature for 1 h under gentle vortexing. Twenty µg of an in house-made anti-elastin antibody (EL) was thiolated with 68 µg Traut's reagent (G-Biosciences, Saint Louis, MO) dissolved in (4-(2-hydroxyethyl)-1-piperazineethanesulfonic acid (20 mM HEPES) buffer (pH=9.0). The mixture was incubated for 1 h at room temperature. Next, the thiolated antibody was added to the PEGylated NPs and conjugated overnight at 4°C under slow rocking.

**EL-PGG-NP injection**. PGG loaded nanoparticles conjugated with elastin antibodies (EL-PGG-NPs) prepared in PBS were injected via the tail vein into AoB rats (10 mg/kg bw) 3 and 4 weeks after AoB surgery. Control rats received elastin antibody-conjugated blank nanoparticles (EL-BLN-NPs).

**Transthoracic echocardiography**. Echocardiography was performed in AoB-PGG or corresponding control rats prior to and after EL-PGG-NP and EL-BLN-NP treatment, respectively, at 3 and 5 weeks after AoB. In brief, rats were anesthetized with isoflurane (1.5% supplemented with O₂ at 2 L/min), eyes were protected with a moisturizing ointment, and temperature and electrocardiograms were continuously monitored. Image acquisition was performed using an MX250 transducer with a 3100 Vevo^{®} imaging system (FUJIFILM VisualSonics, Amsterdam, Netherlands). LV FS was evaluated from the time motion display (M-mode) and LV EF was evaluated from the 2D ultrasound image display (B-mode) acquired in the parasternal long-axis (PLAX) view (Figure 6a). PA imaging was performed in a modified PLAX view obtained by B-mode shifting of the transducer probe toward the RV outflow tract (Figure 6c). PA flow was assessed by pulsed-wave Doppler imaging. TAPSE was assessed in the M-mode by measuring the distance of tricuspid annular movement between end-diastole and end-systole in the mid-esophageal fourchamber view. The aortic arch view was used to control correct clip placement on the aorta and to record flow profiles in the ascending and descending aorta by pulsed-wave Doppler imaging and to acquire M-mode images from the transverse section of the PA (Figure 6b). PA dimensions (maximum and minimum diameter of the pulmonary trunk) and pulmonary arterial blood flow velocity characteristics (PAT and PET) were analyzed using Vevo LAB (FUJIFILM VisualSonics) analysis software. Pulmonary arterial radial strain was calculated as PA RS = (DMax-DMin)/DMin, where DMax and DMin are the maximum and minimum PA diameters measured in the modified PLAX view, respectively.

**Cardiac catheterization and hemodynamics**. Animals were anesthetized with ketamine/xylazine, tracheotomized and ventilated with room air as described above. Following a median thoracotomy, the pericardium was opened, and LVSP followed by RVSP were measured using a microtip Millar catheter (PowerLab, ADInstruments) through the heart apex.

**Heart weight.** Ventricular hypertrophy was assessed as the weight of the left ventricle (including the septum) and right ventricle normalized to bw.

***Ex vivo* uniaxial tensile test**. Similar to human PA samples, the circumferential tensile properties of rat PAs were assessed on a MyoDynamics Muscle Strip Myograph System. Freshly isolated pulmonary trunks were prepared in PBS and dissected into 2-mm-wide rings. Rings were mounted onto the material testing system with the help of hooks. Samples were preconditioned by 5 extension-relaxation cycles (5-10 mN each) and then equilibrated at the baseline pretension of 5 mN. An automated displacement of 5 mm maximal length at a rate of 0.5 mm/s was applied, and parameters of PA biomechanics, namely, *k₁*, *k*₂, and εTrans, were derived from force-displacement curves as described above.

### Histology and microscopy

Histology. Human or rat PA samples were cryo-embedded in OCT Compound (Tissue-Tek) and sliced into 10 µm transverse sections on a Microm HM560 cryostat. Slides were stored at -20°C. Before staining, slides were thawed in PBS and fixed with 4% paraformaldehyde (Alpha Aesar, Thermo Fisher Scientific). Elastic and collagen fibers were visualized with EVG staining (Elastic Stain Kit, ab 1506667, Abcam) according to the manufacturer's protocol. For PGG detection, 10-µm frozen OCT-embedded sections were mounted on positively charged glass slides and rinsed with tap water for 5 min to remove OCT. Sections were stained with 15% FeCl₃ (Sigma-Aldrich) solution in deionized water for 7 min, washed, and observed directly by light microscopy.

**Bright-field microscopy.** Bright-field imaging was performed on an Axioscope 40 microscope (Zeiss) with an Axiocam 506 color camera (Zeiss) and recorded using ZEN 2 (blue edition) software.

**Scanning confocal microscopy.** An A1Rsi+ confocal microscope (Nikon) and NIS-Elements imaging software were used to detect elastic lamellae by autofluorescence (488 nm/0.66 mW laser).

**Transmission electron microscopy**. Samples were fixed immediately with 2.5% glutaraldehyde in 0.1 M sodium cacodylate buffer (both Serva) for 30 min at RT and stored at 4°C. Postfixation was performed with 1% osmium tetroxide (Electron Microscopy Sciences) and 0.8% potassium ferrocyanide II (Roth) in 0.1 Mol/L cacodylate buffer for 1.5 h followed by the dehydration of the samples in a graded ethanol series and the embedding of the samples in Epon resin (Roth). Finally, ultrathin sections with a thickness of 70 nm were stained with uranyl acetate and lead citrate. Samples were examined using a Zeiss EM 906 electron microscope at 80-kV acceleration voltage (Carl Zeiss).

**Image analyses**. EVG staining was quantified based on positively stained area on single-plane images using Fiji-lmageJ.

### Western blotting

Patient samples were powdered in liquid nitrogen, and tissue lysates were prepared in NP-40 buffer (300 mM NaCl, 100 mM Tris pH 8.0, 1% Triton X, 1 tablet protease inhibitor per 10 mL). Sample loading was normalized to protein content measured by a bicinchoninic acid assay (BCA Protein assay kit, Pierce, Rockford, IL), and 25 µg of protein was loaded for each sample on 8-10% SDS- PAGE gels. After electrophoresis, proteins were transferred to 0.2 µm nitrocellulose (1620112, Bio Rad) membranes. Protein transfer was controlled by membrane staining with Ponceau S Staining Solution (59803, Cell Signaling Technology). Membranes were blocked in 3% dried milk (8076.3, Roth) for 30 min at RT and then stained at 4°C overnight with the following primary antibodies at a dilution of 1:1,000: anti-α-elastin (ab21607, 68 kDa, Abcam) or anti-fibrillin-1 (ab124334, 27 kDa, Abcam). Next, membranes were washed 3 times for 5 min each with TBST (20 mMol/L Tris-HCI [pH 7.4], 150 mMol/L NaCl, 0.1% Tween-20) and incubated for 1 h at RT with either of the following secondary antibodies at a dilution of 1:10,000: goat anti-rabbit horseradish peroxidase (HRP) (sc-2004). After washing with TBST 3 times, the immunoreactive bands were detected by a chemiluminescence and fluorescence imager (Celvin^{®} S, Biostep). The quantification of protein bands was performed using ImageJ Lab software, and signals were normalized to β-actin or Ponceau S as loading controls.

### Data analyses and statistics

Force-displacement and stress-strain curves are shown as the mean±standard error of the mean (SEM). The rest of the data are presented as box-and-whisker plots overlaid with dot plots and provide information on the median, lower and upper 25% quartiles; minimum and maximum measurements; and outliers if applicable. Statistical analyses and data visualization were performed using GraphPad Prism 7, OriginPro 8, Microsoft Excel 2016 respectively.

For statistical comparison of two groups, the nonparametric Mann-Whitney U test and the Wilcoxon matched-pairs signed rank test were used for unpaired and paired samples, respectively. For multiple group comparisons, Kruskal-Wallis one-way analysis of variance (ANOVA) on ranks followed by pairwise multiple comparisons (Dunn's test) was applied. The relationship between two variables was evaluated by a two-tailed nonparametric Spearman's rank correlation coefficient. All p-values for statistically significant differences (p<0.05) are shown in figures (or corresponding figure legends or tables).

### Results:

### Conduit PAs are stiffened in PH-LHD

To compare PA biomechanical properties between LHD and healthy controls (donor hearts), circumferential uniaxial tensile tests were performed ex vivo on conduit PA samples obtained from donors and recipients during cardiac transplantation (for the underlying disease of the LHD cohort and age and sex distribution, see Table 1). PH-LHD patients were differentiated from LHD w/o PH patients by a mean PAP ≥ 25 mm Hg and a pulmonary capillary wedge pressure (PCWP) ≥ 15 mm Hg (Table 2). In comparison to LHD w/o PH patients, the PH-LHD cohort had a significantly increased transpulmonary gradient (TPG) and pulmonary vascular resistance (PVR) and a reduced cardiac index (CI) (Table 2).

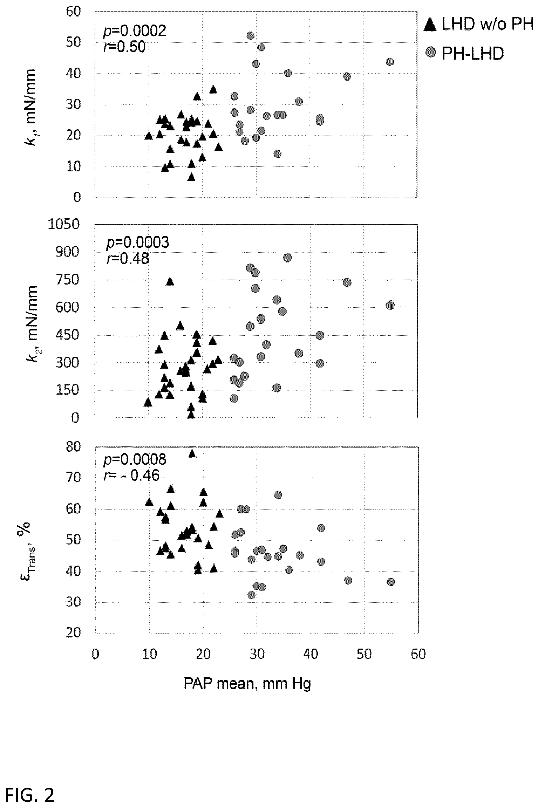

Since arterial mechanics are primarily determined by elastic and collagen fibers, the arterial wall can be structurally viewed as a two-phase material. Accordingly, arterial force-displacement curves sequentially reveal a low-energy toe region and a high-energy linear region reflecting the biomechanical properties of the elastin- and collagen-enriched phases, respectively (Figure 1A). Compared to donor and LHD w/o PH subjects, the averaged force-displacement curve of PH-LHD patients was steeper in both the toe region and the linear region (Figure 1B). Quantitative analysis revealed significant increases in *k₁* and *k*₂, and a decrease in εTrans in PH-LHD patients compared to donors and, to LHD w/o PH patients (Figure 1C), suggesting that altered biomechanical PA characteristics were related to both a load-bearing shift from elastin to collagen and a greater stiffness of elastic and collagen-dominant material in PH-LHD patients. Spearman analyses yielded significant correlations between PA biomechanical properties and mean PAP (Figure 2), consolidating the close association between PA stiffness and pulmonary hemodynamics in PH-LHD.

### Progressive fragmentation and degradation of elastic fibers

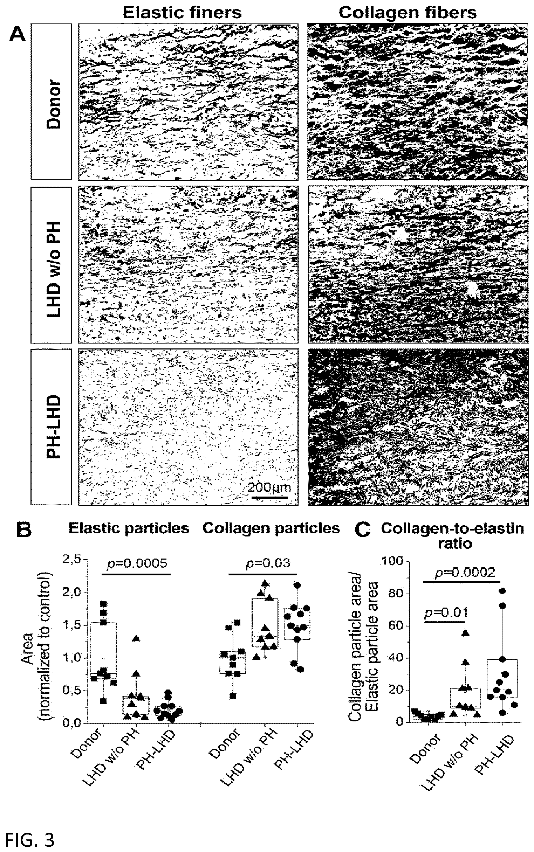

Biomechanical analyses proposed, remodelling of elastic and collagen matrices in the PA wall as a likely cause of vascular stiffening in PH-LHD. To explore this hypothesis, we visualized elastic and collagen fibers on histological PA sections by Verhoeff's elastin Van Gieson (EVG) staining. Relative to healthy controls, extensive ECM remodelling was evident in LHD w/o PH samples but was even more pronounced in PH-LHD samples (Figure 3A). While collagen staining was increased, elastic fiber staining was decreased in LHD w/o PH samples and even more so in PH-LHD samples (Figure 3A-B), resulting in a progressive increase in the collagen-to-elastin ratio (Figure 3C). Transmission electron microscopy (TEM) of the PA media revealed thinner elastic fibers with delaminated fragments in LHD w/o PH samples compared to control samples, while elastic fibers in PH-LHD samples were largely deteriorated and fragmented (Figure 4A, white arrows). In both LHD w/o PH and PH-LHD samples, the elastin core of the elastic fibers was substantially degraded (Figure 4A, black arrows), although the expression levels of α-elastin or fibrillin-1 were unchanged (Figure 4B). These findings indicate increased fragmentation and degradation of elastic lamellae in LHD w/o PH, that further progresses in PH-LHD PAs.

### Elastin stabilization improves human PA biomechanics ex vivo

Since elastic fiber fragmentation in conduit PAs emerged as an early event in LHD that preceded changes in pulmonary hemodynamics and vascular biomechanics, therapeutic targeting of arterial elastin may present a promising modality to prevent PA stiffening and alleviate reactive PH. PGG is an elastin-stabilizing polyphenolic compound that has been proven beneficial in abdominal aortic aneurysm (AAA) animal models. To test whether the stabilization of elastic fibers by PGG may also rescue PA biomechanics, we cultured human PAs ex *vivo* in the presence or absence of 0.1% PGG and induced elastolysis by incubation with 1 U porcine elastase for 24 h. Elastic fibers were almost completely lost by elastase treatment but partially preserved in the presence of PGG (Figure 5A). Importantly, PGG also rescued the biomechanics of elastase-treated PAs, as demonstrated by increased εTrans in uniaxial tensile tests, and even increased the load borne by elastic fibers in naïve PAs (Figure 5B). Thus, PGG is suitable to improve PA biomechanics in pathological scenarios of increased elastolysis, such as PH-LHD.

### Targeted delivery of PGG attenuates PA stiffening and PH in a rat model of PH-LHD

To test this notion, we investigated the therapeutic potential of elastin stabilization by PGG in an established rat model of PH-LHD subsequent to supracoronary aortic banding (AoB) (Figure 6A-B). To specifically target PGG to damaged elastin fibers in vivo, we intravenously administered PGG-loaded NPs conjugated with an elastin antibody (EL-PGG-NPs) to rats at weeks 3 and 4 after AoB, i.e., at a time point when PH-LHD had already been established (Figure 6A; AoB-PGG group). Effective delivery of EL-PGG-NPs to the PA (Figure 6C) and restored - at least in part - the normal structure and crimping of elastic fibers in the PA wall (Figure 6D) was confirmed at week 5 post mortem, by polyphenol-specific histological FeCl3 staining and fluorescent detection of elastic lamellae respectively.

PA biomechanical competences in rat model of PH-LHD were tested ex vivo by uniaxial tensile test. Force-displacement curves revealed significant PA stiffening (with increased *k₁* and *k₂* and reduced εTrans) at 3 and 5 weeks post AoB (Figure 7A-B). EL-PGG-NP treatment at large normalized PA tensile properties as determined by analysis of force-displacement curves (Figure 7A (5w) and Figure 7B). Importantly, beneficial effects of treatment with EL-PGG-NPs were also evident when comparing 5-week EL-PGG-NP-treated AoB rats vs. 3-week untreated AoB rats, demonstrating that PGG not only prevented progression but also reversed vascular stiffening in this model of PH-LHD.

Then we addressed the effect of EL-PGG-NP treatment on pulmonary hypertension. Compared to sham-operated animals, AoB rats developed increased left ventricular systolic pressure (LVSP) and left ventricular hypertrophy (measured as (LV+Sw)/Bw) 1 week post AoB and increased right ventricular systolic pressure (RVSP) and right ventricular hypertrophy (quantified as RVw/Bw) 3 weeks post AoB (Figure 8). At week 5 post AoB, AoB-PGG animals had a similar increase in LVSP and LV hypertrophy as vehicle-treated AoB rats (AoB-BLN); however, RVSP and RV hypertrophy were markedly reduced (albeit just missing the level of significance in the case of RV hypertrophy) and did not differ from corresponding values in sham controls (Figure 8).

This notion was further supported by longitudinal transthoracic echocardiography (Figure 9). In line with progressive LV failure, left ventricular fractional shortening and left ventricular ejection fraction (LV FS and LV EF, respectively, Figure 9A,D) were decreased in both elastin antibody-conjugated NPs lacking PGG (EL-BLN-NP)- and EL-PGG-NP-treated rats at 5 weeks post AoB compared to pretreatment values at 3 weeks post AoB. In EL-BLN-NP rats, PA distensibility (assessed as PA radial strain (PA RS); Figure 9B,D) and tricuspid annular plane systolic excursion (TAPSE, Figure 9D) decreased in parallel, indicating progressive PH-LHD, while the pulmonary acceleration time (PAT) and PAT to pulmonary ejection time (PET) ratio (PAT/PET, Figure 9C,D) remained largely unchanged. In contrast, EL-PGG-NP-treated rats showed an increase in PA distensibility and PAT, and a stabilization of TAPSE over the same time interval. As such, PGG treatment improved PA biomechanics and pulmonary hemodynamics even in the face of progressively deteriorating LV function.

### Discussion:

In this study, we identified impaired PA biomechanical competences as a hallmark of PH-LHD. PA stiffening in PH-LHD is associated with a dysregulation of ECM: loss of elastin fibers, and increased collagen abundance. Importantly, these changes already emerge in the PAs of LHD patients, suggesting that PA remodelling processes are already initiated prior to PH onset, ultimately causing PA stiffening. The pathophysiological relevance of this process is evident from preclinical experiments in which elastin stabilization normalized PA biomechanics as well as pulmonary and RV hemodynamics despite persistent LV failure. PA biomechanics thus emerge as an important pathophysiological process that may serve as both a prognostic biomarker and a therapeutic target in PH-LHD.

### Elastic fiber remodelling in the PA media is evident in LHD patients prior to the onset of PH and further progresses to PA stiffening in PH-LHD patients

In healthy arteries, elastic fibers facilitate arterial compliance by straightening in response to load and recoiling in the unloaded state. To this end, elastic fibers are organized into continuous and tortuous lamellae that are circumferentially arranged in the arterial wall. Here, changes in elastic fiber composition could be detected in the PA wall of LHD patients even prior to the onset of PH or altered PA biomechanics. Specifically, microscopic analyses revealed elastic fiber fragmentation and degradation. In contrast to LHD w/o PH, the PAs of PH-LHD patients displayed altered circumferential tensile properties. Specifically, ex *vivo* measured force-displacement curves revealed steeper slopes of both the toe region and the linear region and a marked shift to lower εTrans. As the toe region reflects the properties of elastin-enriched material, these findings suggest a loss of load-bearing elastic fibers with an increased stiffness and/or straightening of the residual elastic fibers. Microscopy confirmed extensive rarefaction in elastic fibers, with a loss of the fiber elastin core at the ultrastructural level.

### Elastic fiber fragmentation - an early marker and potential mechanism of PA remodelling

In the present study, we identified the fragmentation and degradation of elastic fibers as an early event in PA remodelling that precedes and likely contributes to PA stiffening and impaired arterial biomechanics in PH-LHD. The functional consequences of elastic fiber degradation may, however, extend beyond pure mechanobiology, as fiber degradation is known to mediate arterial remodelling processes, specifically, attachment to elastic fibers is important to maintain SMCs in their contractile phenotype and prevent SMC migration. Elastic fiber fragmentation also releases bioactive elastin-derived peptides (EDPs) that act through the membrane-bound elastin receptor complex and regulate a series of biological processes. Although the role of EDPs was not a focus of our current study, it is tempting to speculate that the observed PA remodelling processes in PH-LHD may be induced or enhanced by EDP-mediated signalling. For example, EDPs can regulate elastic fiber degradation through a positive feedback mechanism by stimulating cytokine signalling and activating inflammatory cells, which in turn secrete proteases to digest elastic fibers. EDPs have also been implicated in the induction of hyperglycemia, and the resulting high glucose levels may fuel the formation of AGEs that mediates ECM cross lining and promotes stiffening. In addition to EDPs, elastic fiber fragmentation may also release autocrine growth factors such as transforming growth factor (TGF)-β, a key driver of lung vascular remodelling. TGF-β bioavailability is regulated by the binding of the latent TGF-β binding protein (LTBP) - latency-associated peptide (LAP) -TGF-β complex to fibrillin, with a consequential release of active TGF-β in response to elastolysis.

As such, elastic fiber fragmentation may constitute an early event as well as a relevant pathophysiological mechanism of PA remodelling. Specifically, elastolysis appears to occur early in left heart failure but may initiate a cascade of remodelling processes, including progressive loss of elastin and increased production of fibrillar collagen, that ultimately cause PA stiffening and PH secondary to LHD. If this is indeed the case, the prevention of elastin degradation may present a promising strategy to attenuate ECM remodelling, improve arterial biomechanics, and potentially reduce PH and RV load.

### Elastic fiber stabilization rescues PA biomechanics and attenuates PH

To test the effect of elastic fiber stabilization on PA biomechanics and PH, we aimed to counteract elastic fiber degradation in PAs. To this end, polyphenols such as PGG and epigallocatechin gallate (ECGC) have shown considerable promise due to their ability to induce elastin synthesis, organization, and crosslinking while concomitantly blocking the activity of elastindegrading enzymes. Accordingly, local application of PGG could effectively decrease elastic fiber degeneration and reduce aneurysmal expansion in a rat AAA model, providing proof-of-principle for the ability of PGG to stabilize elastic fibers and improve arterial biomechanics *in vivo.* This approach was recently further refined by PGG loading into EL-PGG-NPs. As these antibodies will preferentially bind to degrading elastin fibers, this system allows specific targeting of PGG to the sites of vascular damage. In the rat AAA model, systemic delivery resulted in specific accumulation of EL-PGG-NPs at the AAA site and proved efficacious to warrant long-term aortic biomechanical stability in vivo, with the inhibition of macrophage infiltration and MMP activity and the restoration of an intact elastic lamina.

As PGG has thus far not been tested on PAs, we first assessed its effects on elastic fibers and arterial biomechanics in human PAs ex vivo. In both naïve PAs and in PAs treated with elastase, PGG increased elastin content and improved arterial biomechanics, as demonstrated by a shift in load-bearing elements from collagen to elastic fibers, further substantiating the therapeutic promise of PGG. Next, we probed for the effects of EL-PGG-NPs in vivo in a rat AoB model that replicates the cardinal features of human PH-LHD, namely, PA stiffening with a shift of load-bearing elements from elastin to collagen, increased RVSP, and RV hypertrophy. Systemic administration of EL-PGG-NPs yielded effective delivery of PGG to the PAs of AoB rats and improved PA biomechanics and RV hemodynamics in vivo. Importantly, and in line with the proposed targeting of EL-PGG-NPs to degrading fibers, this approach proved beneficial in a therapeutic setting, i.e., when EL-PGG-NPs were delivered at a time point when not only LHD but also PA stiffening, PH and RV hypertrophy were evident. Moreover, EL-PGG-NPs not only prevented disease progression but also partially reversed impaired PA biomechanics and, as such, PH and RV hypertrophy. Specifically, EL-PGG-NP treatment right-shifted the transition from more elastin- to collagen-dominated mechanical competence εTrans in PA force-displacement curves, indicating a reverse shift in load-bearing elements from collagen back to elastin. In line with this interpretation, EL-PGG-NP treatment also increased PA radial strain in vivo. The rescue of PA biomechanics was associated with improved RV hemodynamics and hypertrophy, highlighting the relevance of PA stiffening in the progression of PH and RV hypertrophy in PH-LHD.

In the present study, we demonstrate considerable PA stiffening in patients with PH-LHD that is associated with a switch in load-bearing elements from an elastin to a collagen fiberdominated tissue and altered ECM composition. Corresponding changes at the transcriptomic and microscopic levels were already evident in LHD patients prior to the onset of PA stiffening and PH, indicating that changes in PA ECM composition constitute an early event and potential pathomechanism in disease progression. In line with this notion, therapeutic stabilization of elastic fibers rescued PA biomechanics and attenuated PH, thus consolidating the pathogenic relevance of PA stiffening for RV hemodynamic alterations and highlighting the therapeutic potential of ECMtargeted interventions for the treatment of pulmonary vascular diseases associated with ECM remodelling and PA stiffening.

### Summary:

In the present study, we performed a comprehensive mechanobiological analysis of pulmonary artery (PA) samples from left heart disease (LHD) patients. We identified PA stiffening as a characteristic feature of pulmonary hypertension secondary to left heart disease (PH-LHD). Extracellular matrix (ECM) remodelling precedes the onset of clinical PH and was characterized by progressive fragmentation and degradation of elastin and a parallel increase collagen. In ex *vivo* cultured human PAs, the stabilization of elastin with the polyphenolic compound pentagalloyl glucose (PGG) reduced elastolysis and improved arterial biomechanical competences. In a rat model of PH-LHD, nanoparticle (NP)-based targeted delivery of PGG reversed PA stiffening and prevented the development of PH, thus pointing toward a core role of ECM remodelling in PH and accordingly, to ECM as a promising therapeutic target in LHD patients.

## Claims

1. Pentagalloyl glucose (PGG) or a pharmaceutically acceptable salt thereof for use in treatment or prevention of pulmonary hypertension; preferably the pulmonary hypertension is secondary pulmonary hypertension; more preferably the pulmonary hypertension is pulmonary hypertension secondary to left heart disease (LHD; WHO group II).

2. Pharmaceutical composition for use in treatment or prevention of pulmonary hypertension, wherein the pharmaceutical composition comprises pentagalloyl glucose or a pharmaceutically acceptable salt thereof and a pharmaceutically acceptable excipient; preferably the pulmonary hypertension is secondary pulmonary hypertension; more preferably the pulmonary hypertension is pulmonary hypertension secondary to left heart disease (LHD; WHO group II).

3. The pharmaceutical composition for use according to claim 2, wherein the pentagalloyl glucose is combined with a delivery vehicle.

4. The pharmaceutical composition for use according to claim 3, wherein the delivery vehicle comprises a microparticle, a nanoparticle, a hydrogel, a perivascular drug delivery vehicle, an endovascular drug delivery vehicle, a stent or a combination thereof.

5. The pharmaceutical composition for use according to anyone of claims 3 to 4, wherein the delivery vehicle comprises a microparticle or a nanoparticle, preferably the microparticle or nanoparticle comprises a peptide, a protein, and/or a polymer.

6. The pharmaceutical composition for use according to claim 5, wherein the microparticle or a nanoparticle is biodegradable.

7. The pharmaceutical composition for use according to claim 5 or 6, wherein the PGG or the pharmaceutically acceptable salt thereof is arranged within and/or is bound to the surface of the microparticle or nanoparticle.

8. The pharmaceutical composition for use according to anyone of claims 3 to 7, wherein the drug delivery vehicle comprises an anchoring agent suitable for targeting of pulmonary blood vessels, preferably for targeting of pulmonary arteries.

9. The pharmaceutical composition for use according to claim 8, wherein the anchoring agent is covalently bonded to the drug delivery vehicle.

10. The pharmaceutical composition for use according to claim 8 or 9, wherein the anchoring agent binds specifically to a structure associated with a pulmonary blood vessel; preferably to a structure of a cell of a pulmonary blood vessel or a component of the extracellular matrix of a pulmonary blood vessel.

11. The pharmaceutical composition for use according to claim 10, wherein the structure associated with a pulmonary blood vessel is elastin, preferably human elastin.

12. The pharmaceutical composition for use according to anyone of claims 8 to 11, wherein the anchoring agent comprises or consists of an antibody or a specific binding fragment thereof.

13. The pharmaceutical composition for use according to claim 12, wherein the anchoring agent is coupled to the delivery vehicle via a linker molecule; preferably via a PEG-linker.

14. The pharmaceutical composition for use according to anyone of claims 3 to 13, wherein the pharmaceutical composition is configured for topical or systemic administration, preferably for intravascular, intravenous, intra-arterial, intracardial, intra-pulmonal and/or nasal administration.
